(19)

**Europäisches Patentamt**
**European Patent Office**
**Office européen des brevets**

(11) **EP 1 465 835 B1**

(12) **EUROPEAN PATENT SPECIFICATION**

(45) Date of publication and mention
of the grant of the patent:
**31.12.2008 Bulletin 2009/01**

(51) Int Cl.:
*C01B 9/04* *(2006.01)*    *C01B 11/20* *(2006.01)*
*C01D 3/10* *(2006.01)*    *C01B 7/09* *(2006.01)*

(21) Application number: **02710244.1**

(86) International application number:
**PCT/IB2002/000386**

(22) Date of filing: **25.01.2002**

(87) International publication number:
**WO 2003/062143 (31.07.2003 Gazette 2003/31)**

(54) **PREPARATION OF NON-HAZARDOUS BROMINATING REAGENTS**

HERSTELLUNG VON UNGEFÄHRLICHEN BROMIERUNGSREAGENZIEN

PREPARATION DE REACTIFS DE BROMATION NON DANGEREUX

(84) Designated Contracting States:
**AT BE CH CY DE DK ES FI FR GB GR IE IT LI LU MC NL PT SE TR**

(43) Date of publication of application:
**13.10.2004 Bulletin 2004/42**

(73) Proprietor: **Council of Scientific and Industrial Research**
**New Delhi 110 001 (IN)**

(72) Inventors:
• **GADDE, R.**
**Cen.Salt and Marine Chemicals Res.Inst.**
**Gujarat 364 002 (IN)**
• **GHOSH,P.K.**
**Cen.Salt and Marine Chemicals Res.Inst.**
**Gujarat 364 002 (IN)**
• **MEHTA, A.S.**
**Cen.Salt and Marine Chemicals Res.Inst**
**Gujarat 364 002 (IN)**
• **SUBBARAYAPPA**
**Cen.Salt and Marine Chem. Res.Inst.**
**Gujarat 364 002 (IN)**
• **JETHVA, A,D**
**Cen.Salt and Marine Chemicals Res.Inst**
**Gujarat 364 002 (IN)**

• **VAGHELA,S,S.**
**Cen.Salt and Marine Chem. Res.Inst.**
**Gujarat 364 002 (IN)**

(74) Representative: **Wenger, Joel-Théophile et al**
**Leman Consulting S.A.**
**Chemin de Précossy 31**
**1260 Nyon (CH)**

(56) References cited:
WO-A-02/057207        DE-A- 2 613 969
US-A- 3 232 959        US-A- 5 817 888
US-A- 6 156 229

• DATABASE WPI Section Ch, Week 199517 Derwent Publications Ltd., London, GB; Class E33, AN 1995-125369 XP002214531 & IL 84 830 A (BROMINE COMPOUNDS LTD), 24 January 1995 (1995-01-24)

Remarks:
The file contains technical information submitted after the application was filed and not included in this specification

**Description**

**TECHNICAL FIELD**

The present invention relates to the preparation of non-hazardous brominating reagents.

The invention particularly relates to suitable mixtures of alkali bromide and alkali bromate salts that can be prepared in stable form from inexpensive raw material and can be used as a substitute for liquid bromine in aromatic bromination reactions and also bromine addition in unsaturated compounds.

**BACKGROUND ART**

Liquid bromine is used to prepare a variety of brominated compounds through addition or substitution reactions. The latter includes commercially important products such as: tetrabromobisphenol-A (TBBPA)—a flame retardant, eosin—a pigment used in personal care products, bromoacetanilide—an analgesic and antipyretic agent, tribromophenol—an intermediate used in the manufacture of antiseptic, germicide, fungicide, fire extinguishing fluids, and fire retardant, and 2-bromo-4-nitro acetanilide—a drug intermediate used in the preparation of nimenslide. Likewise, there are a number of addition compounds of bromine that have utility as intermediates or products. However, liquid bromine is hazardous by nature and requires extreme care in its production, transportation, and utilization. Moreover, for substitution reactions depicted by equation 1, half of the bromine atoms end up in the effluent as hydrobromic acid.

$$R\text{-}H + Br_2 \rightarrow RBr + HBr \qquad (1)$$

Bromine atom efficiency of liquid bromine can be 100 % for addition across olefins (equation 2) but the need to handle the hazardous liquid bromine remains.

$$R\text{-}CH=CH_2 + Br_2 \rightarrow RCHBrCH_2Br \qquad (2)$$

Z. E. Jolles (Bromine and its compounds, Ernest Benn Limited London **1966**, p352) describe the preparation of a number of dibromo compounds through addition of liquid bromine across unsaturated organic compounds. Such products are obtained in high yields but an important disadvantage is the hazards of handling liquid bromine.

C. A. Buechler and D. E. Pearson, (Survey of organic syntheses Wiley -Inter science, New York 1970 Chapter7) have reported the preparation of tribromophenol using liquid bromine as brominating agent. The drawbacks of this method are that it requires special devices for handling corrosive liquid bromine and at least half of the bromine atoms in the reagent end up in the effluent in the form of hydrobromic acid.

Reference may be made to S. Armstrong in US 5,475,153, Dec. 12, 1995 who brominated Bisphenol-A with liquid bromine to get 98 % pure tetrabromobisphenol-A (m.p, 180 °C) and where hydrogen peroxide was combined with the reactants to reuse the HBr produced as in equation 1 and thereby reduce the amount of bromine required. However, the principal difficulty of the hazardous nature of liquid bromine remains.

Brominating agents that are easy to handle are known but are used mainly for more selective transformations or those where bromine is less effective. A. Groweiss in Organic Process & Development 2000, 4, 30-33, described the use of sodium bromate for bromination of aromatic compounds that contain deactivating substituents such as nitrobenzene, benzoic acid, benzaldehyde, 4-nitrofluorobenzene and 4-fluorobenzoic acid. In this process, the addition of a strong acid such as sulfuric acid into a stirred aqueous solution or slurry of the substrate and stoichiometric quantity of bromate salt at 40-100 °C, leads to the decomposition of the bromate ions and production of active brominating species. The drawback of sodium bromate is that it is costly and its use cannot be justified in more conventional bromination reactions that can be effected by liquid bromine as such.

Z. E. Jolles (Bromine and its compounds, Ernest Benn Limited London **1966**, p394) has added a mixture of 356 g (2 moles) of N-bromosuccinimide and 4 g (0.0165 moles) of benzoyl peroxide over 20 min to a solution of 220 g (2.24 moles) of 3-methyl thiophene and 4 g (0.0165 moles) benzoyl peroxide in 700 ml of dry benzene under stirring at reflux conditions. After all the succinimide is added, the reaction mixture is cooled to below 5°C. The benzene is distilled at 75-78°C under reduced pressure to give 280 g of.3-bromomethyl- thiophene. Although N-bromosuccinimide is a useful reagent for specific bromination reactions, it is a costly brominating agent and its use cannot be justified in those bromination reactions where liquid bromine would suffice; even more so since its preparation involves use of liquid bromine in any case (N-bromosuccinimide is prepared by reacting succinimide with bromine liquid below 0 °C in potassium hydroxide solution).

P. C. Merker and J. A. Vona (J Chem. Ed. 1949, 26, 613) prepared *p*-bromoacetanilide by reacting 31.5 g (0.232 mole) of acetanilide in 50 ml of glacial acetic acid with 38 g (0.119 mole) of pyridinium bromideperbromide in hot 40 ml glacial acetic acid. The mixture was allowed to stand at room temperature for 30 min. To it, 2 ml of saturated sodium bisulfite solution was added. The resulting mass was filtered, washed with water and finally recrystallized from hot 95% aqueous ethanol to yield 13 g of p-bromoacetanilide having m.p. 168 °C. The drawbacks of this method are that the brominating agent requires liquid bromine and hydrobromic acid in its preparation (L. F. Fieser and M. Fieser, Reagents for Organic Chemistry Vol. 1, John Wiley, New York, 1967, p967) and the reagent is costlier than liquid bromine.

G. Rothenberg and J. H. Clark (Organic Process & Development 2000, 4, 270-274) have claimed the catalytic bromination of various aromatic compounds using an alkali bromide or hydrobromic acid and hydrogen peroxide in the presence of 1-2 mol % vanadium pentoxide catalyst. The drawbacks of this method are that more than stoichiometric quantities of hydrogen peroxide are required and the reaction needs a catalyst.

G. Ramachandraiah, P. K. Ghosh, A. S. Mehta, R. P. Pandya, A. D. Jethva, S. S. Vaghela, S. N. Misra (pending US Pat. Appln. No. 09/767,667 [2001]) have prepared tetrabromobisphenol-A from bisphenol-A using 2:1 molar ratio of bromide and bromate salts as brominating agent. To 0.50 kg (2.19 moles) of bisphenol-A in 1.50 liters of methylene chloride, a solution of 0.63 kg (6.14 moles) of sodium bromide, 0.44 kg (2.93 moles) of sodium bromate and 1 g of sodium lauryl sulfate in 2.5 liters of water was added. The flask was cooled to 10 °C by placing it in a cold water bath. To it, 0.90 liters (10.8 moles) of 12 N hydrochloric acid was added over 3 h under stirring. The contents were stirred for another 0.5 h and the separated solid product was filtered, washed twice with deionized water and dried in oven at 100 °C to give a yield of 0.85 kg of TBBPA. The organic layer was recycled in subsequent batches. The isolated yield of TBBPA (m.p. 178-180 °C) over three batches was 85.4 %. Although the method has several advantages in that the brominating reagent is easy to handle, no catalyst is required, the bromine atom efficiency for the aromatic substitution reaction studied is as high as 95-100 %, the main drawback of this method is that alkali bromide and bromate salts are individually much costlier than liquid bromine. Moreover the 4:2 stoichiometry of bromide:bromate is suitable for substitution reactions but not for addition of bromine across double bonds. According to the present invention, the main drawback of pending US Pat. Appln. No. 09/767,667 (2001) has been overcome by using a mixture of alkali bromide and alkali bromate salts of the desired ratios that can be prepared inexpensively from the intermediate of bromine recovery plants, the said mixture being easy to handle and stable under storage while, under the conditions of bromination reaction, the bromide and bromate salts self annihilate one another to create reactive species of bromine that are useful in the safe preparation of several organo bromine compounds as demonstrated through working examples.

## DISCLOSURE OF THE INVENTION

The main object of the present invention is the preparation of suitable mixtures of alkali bromide and alkali bromate salts that are easy to handle, stable on storage, and can replace corrosive liquid bromine in bromination reaction.

Another object of the invention is to prepare a non-hazardous brominating reagent from an aqueous alkaline bromine byproduct solution obtained from bromine recovery plant.

Another object of the present invention is to utilize the intermediate of bromine recovery plants that are based on the "Cold Process" which typically contains 25-35 % (w/v) "bromine" dissolved in lime or sodium hydroxide.

Yet, another object of the present invention is to alter the composition of the industrial alkaline bromine mixture to maximize bromine atom efficiency in the reactions of equations 1 and 2 and minimize discharge in effluent.

Yet, another object of the present invention is to adjust appropriately the bromide:bromate ratio of the intermediate by adding alkali bromide salt to achieve a precise stoichiometry of 5:1 bromide:bromate suitable for equation 2.

Yet, -another object of the present invention is to adjust appropriately the bromide:bromate ratio of the intermediate by adding alkali bromate salt to achieve a stoichiometry of 2:1 bromide:bromate suitable for equation 1.

Yet, another object of the present invention is to adjust appropriately the bromide:bromate ratio of the intermediate by utilizing inexpensive oxidizing agents such as sodium hypochlorite that can oxidize bromide ion to bromate ion to achieve a stoichiometry of 2:1 bromide:bromate suitable for equation 1.

Yet another object of the present invention is to make the brominating reagent in the desired physical form.

Yet another object of the present invention is to activate the brominating agent with a suitable acid during bromination reactions of organic substrates.

## SUMMARY OF THE PRESENT INVENTION

Accordingly, the present invention provides an non-hazardous brominating reagent from an aqueous alkaline bromine byproduct solution obtained from bromine recovery plant and containing 25 to 35 % bromine dissolved in aqueous lime or sodium hydroxide containing alkali bromide and alkali bromate mixture having bromide to bromate stoichiometric ratio in the range of 5:1 to 5.1:1 or 2:1 to 2.1:1 and a pH ranging between 8-12. This invention also provides a process for producing the above said brominating agent and use of said brominating agent for brominating organic substrate.

**DETAILED DESCRIPTION OF THE INVENTION**

Accordingly, the present invention provides a stable non-hazardous brominating reagent from an aqueous alkaline bromine byproduct solution obtained from bromine recovery plant, said bromine byproduct solution containing 25 to 35 % bromine dissolved in aqueous lime or sodium hydroxide in the form of alkali bromide and alkali bromate mixture, said reagent having a pH ranging between 8.5 - 10.5 and containing 100 - 350 g/L bromine or in a solid form having 45-55% (w/w) bromine.

In an embodiment of the invention relates to a cost-effective process for the preparation of a stable and non-hazardous brominating reagent which comprises treating aqueous alkaline bromine containing 25-35 % bromine dissolved in aqueous lime or sodium hydroxide as an alkali bromide and alkali bromate mixture at a pH ranging between 8-12 with alkali bromide to increase the stoichiometric ratio of bromide to bromate in the range of 5.1:1 to 5:1 or adding an oxidizing agent to decrease the stoichiometric ratio of bromide to bromate in the range of 2.1:1 to 2:1 to obtain the desired brominating reagent solution having pH ranging between 8.5-10.5 and containing 100-350 g/L bromine, optionally evaporating the above said brominating reagent solution to obtain the desired brominating reagent in a solid form possessing 45-55 % (w/w) bromine.

In another embodiment of the invention, wherein the bromide to bromate ratio is increased to 5:1 by adding appropriate quantity of alkali bromide salt to the aqueous alkaline bromine solution.

In still another embodiment of the invention, wherein the bromide to bromate ratio of 2:1 is achieved by treating the aqueous alkaline bromine solution with 1-10% concentration of aqueous sodium hypochloride for a period of 6-24 hours at a temperature in the range of range of 25-30°C and more preferably by adding alkali into the aqueous alkaline bromine solution followed by passing chlorine gas to generate hypochloride in situ to convert bromide salt to bromate salt by an oxidation process.

Yet another embodiment of the invention, wherein the bromide to bromate ratio of alkaline bromine mixture is decreased by adding appropriate quantity of alkaline bromate salt, preferably sodium or calcium bromate while ensuring that pH of the solution remains between 8-10 and there is no rise in temperature.

Yet another embodiment of the invention, wherein the solution of brominating reagent is evaporated by conventional techniques, preferably in solar pans for large scale production to obtain solid brominating reagent which is finally ground to get a homogenous mixture of salts having bromine content in the range of 90-100%.

Yet another embodiment of the invention, wherein the said brominating reagent is activated *in situ* during the bromination reaction through addition of stoichiometric quantity of a mineral acid, preferably hydrochloric acid.

One more embodiment of the invention provides a method for brominating aromatic compounds by using brominating reagent as claimed in claim 1, wherein the aromatic compound used is selected from group consisting of bisphenal A, bromophenol and olefin selected from styrene and cyclohexene and other class of aromatic compounds, the said method comprising: treating aqueous alkaline bromine containing 25-35 % bromine dissolved in aqueous lime or sodium hydroxide as an alkali bromide and alkali bromate mixture at a pH ranging between 8-12 with alkali bromide to increase the stoichiometric ratio of bromide to bromate in the range of 5.1:1 to 5:1 or adding an oxidizing agent to decrease the stoichiometric ratio of bromide to bromate in the range of 2.1:1 to 2:1 to obtain the desired brominating reagent solution having pH ranging between 8.5-10.5 and containing 100-350 g/L bromine, optionally evaporating the above said brominating reagent solution to obtain the desired brominating reagent in a solid form possessing 45-55 % (w/w) bromine with active bromine content that is 90-100 % of total bromine content and activating the brominating agent *in situ* during bromination reaction by adding 9-12 ml of 12N hydrochloric acid into the reaction vessel containing 1-3g of organic substrate and 1-20 g of said brominating reagent or alternatively by adding 1-20 g of brominating reagent into the reaction vessel containing 1-3 g of organic substrate and 9-12 ml of 12N hydrochloric acid.

The present invention provides a cost-effective process of preparation of a stable and non-hazardous brominating reagent containing 5:1 to 2:1 stoichiometric ratio of alkali bromide: alkali bromate which comprises treatment of aqueous alkaline bromine containing 25-35 % bromine dissolved in aqueous lime or sodium hydroxide as an alkali bromide/alkali bromate mixture in the pH range 8-12 with an appropriate quantity of alkali bromide to increase bromide: bromate ratio or with an oxidizing agent to decrease bromide/bromate ratio, as appropriate, yielding the desired brominating reagent solution with pH 8-10 and containing 100-350 g/L bromine, optionally evaporating the solution to yield a solid form of brominating agent possessing 45-55 % (w/w) bromine with active bromine content that is 90-100 % of total bromine content, and activating the brominating agent *in situ* during bromination reactions by controlled addition of appropriate quantity of mineral acid into the reaction vessel containing organic substrate and brominating reagent or alternatively through controlled addition of brominating reagent into reaction vessel containing organic substrate and mineral acid.

In an embodiment of the present invention, the alkaline bromine having 29.3% (w/v) bromine dissolved in sodium hydroxide (37.2 °Be, pH 8.73) and 28.1% (w/v) bromine dissolved in lime (37.2 °Be, pH 10.25) are obtained from bromine recovery plants based on "Cold Process" technology.

In another embodiment of the present invention, alkaline bromine may be obtained from other sources than bromine recovery plants based on "Cold Process" technology.

In yet another embodiment of the present invention bromide salt is added to the alkaline bromine having bromide to bromate ratio less than 5:1 to adjust the bromide to bromate ratio to 5:1.

In yet another embodiment of the present invention, the alkaline bromine is reacted with required quantity of sodium hypochlorite in a closed vessel over a period of 6-24 h to obtain the brominating reagent with 2:1 ratio of bromide to bromate.

In yet another embodiment of the present invention, the alkaline bromine may be mixed with required quantity of a bromate salt over a period of 10-30 min to obtain the brominating reagent with 2:1 stoichiometry of bromide:bromate.

In yet another embodiment of the present invention, the reaction/mixing is conducted typically in the temperature range between 25-30 °C.

In yet another embodiment of the present invention, the treated alkaline bromine solutions containing 5:1 and 2:1 bromide to bromate are used as reagents for bromine addition and substitution reactions, respectively.

In yet another embodiment of the present invention, the brominating reagent solution is evaporated and dried to obtain a solid, which can be used as brominating reagent.

In yet another embodiment of the present invention, the brominating agent can be activated during bromination reactions addition of a suitable acid.

In yet another embodiment of the present invention the acid used during the bromination reaction is preferably an inexpensive mineral acid and more specifically an acid which produces soluble calcium salts which is advantageous when the brominating agent is based on alkaline (lime) bromine and/or calcium hypochlorite.

Bromine is manufactured commercially by the "Steaming Out" and "Cold" Processes. In the latter process, bromine liberated from the feed by sparging with chlorine gas is initially trapped in aqueous solution in concentrated form wherein it reacts with the alkali and disproportionate as per the reaction of equation 3 to produce five parts of bromide ion and one part of bromate ion. The medium is then acidified to re-liberate bromine as per the reaction of equation 4. This bromine can then be collected.

$$3Br_2 + 6OH^- \rightarrow 5Br^- + BrO_3^- + 3H_2O \qquad (3)$$

$$5Br^- + BrO_3^- + 6H^+ \rightarrow 3Br_2 + 3H_2O \qquad (4)$$

whereas, the mixture of bromide and bromate is unstable under acidic conditions, it is stable over many months under alkaline conditions. It has been found in the course of this invention that it is possible to apply the knowledge of equation 4 and convert the product mixture of equation 3 into a reagent to carry out the reaction of equation 2 as shown below:

$$5Br^- + BrO_3^- + 6H^+ \rightarrow [3Br_2] + 3H_2O \qquad (5a)$$

$$R\text{-}CH{=}CH_2 + [Br_2] \rightarrow RCHBrCH_2Br \qquad (equation\ 2)$$

Care must, however, be taken to ensure that sufficient time is given for equation 5a so that molecular bromine can be generated prior to the reaction of equation 2. Otherwise, tendency to form side products is higher as demonstrated later in the examples.

The reagent can also be used to carry out equation 6, as described in pending US Pat. Application No. 09/767,667 (2001), when the reaction mixture containing such brominating reagent and organic compound is acidified whereupon active bromine is generated. Alternatively, the brominating agent can be added into a solution of the organic compound and desired quantity of acid.

$$3R\text{-}H + 5Br^- + BrO_3^- + 6H^+ \rightarrow 3RBr + 3HBr + 3H_2O \qquad (6)$$

Pending US Pat. Appln. No. 09/767,667 (2001) states that the bromide/bromate stoichiometric ratio should be 4:2 instead of 5:1 for maximum bromine atom efficiency, as illustrated by equation 7.

$$6R\!\!-\!\!H + 4Br^- + 2BrO_3^- + 6H^+ \rightarrow 6RBr + 6H_2O \qquad (7)$$

It occurred to us in the course of the invention that the desired bromide:bromate stoichiometric ratio can be achieved in cost-effective manner as shown by equations 8 and 9. Hypochlorite is generated as in equation 8 and used in the reaction of equation 9 to attain the desired 4:2 stoichiometry of bromide:bromate.

$$3Cl_2 + 6\ OH^- \rightarrow 3ClO^- + 3Cl^- + 3H_2O \qquad (8)$$

$$5Br^- + BrO_3^- + 3ClO^- \rightarrow 4Br + 2BrO_3^- + 3Cl^- \qquad (9)$$

The preparation of hypochlorite involves handling alkali and chlorine, both of which are used by manufacturers of bromine by the "Cold Process". Moreover, hypochlorite is among the cheapest oxidizing agents, which can effect the desired oxidation reaction of equation 9, and, therefore, is an ideal choice although other oxidizing agents could also be used.

The reaction of equation 9 is carried out in the laboratory in a stoppered 0.25-5.00 L round bottom flask. Alkaline bromine containing 3-265 g of dissolved bromine in lime or sodium hydroxide was mixed with calculated amount 0.8-63.0 g of sodium hypochlorite under thorough stirring. The reactants were allowed to react for 6-24 h to obtain the desired brominating reagent in solution form. The temperature of the vessel during the reaction was in the range of 27-30 °C. It is advisable to conduct the reaction of sodium hypochlorite with alkaline bromine in a closed vessel for at least 12 h.

In the second method, the alkaline bromine containing 3-265 g of dissolved bromine was mixed with a solution of calculated amount (1.5-118 g) of sodium bromate in a 0.25-5.00 L flask to give liquid brominating reagent or solid on evaporation and drying of it under vacuum.

Better brominating reagent for equation 7 is obtained if the bromide:bromate ratio is maintained between 2.0-2.1. Brominating agent with bromide:bromate > 2.1 leads to decrease in bromine atom efficiency whereas undesired products tend to be produced when bromide:bromate <2.

In the preferred form of the invention, the aqueous brominating agent with pH in the range 8.5-10.5 and the desired stoichiometry of bromide:bromate was evaporated on a steam bath and dried under vacuum to get the reagent in solid form. Bromine manufacturers generally have access to solar evaporation ponds that can be used to produce the solid reagent cost-effectively on large scale. Apart from the advantage of higher bromine content, such form of the reagent would be easier and cheaper to transport than the solution.

The brominating reagent (solid and solution) was characterized by determining its bromate and bromide contents. 1 g of solid or 1 ml of liquid brominating reagent was dissolved/diluted with water to a volume of 100 ml and used as stock in the estimation of bromate and bromide. To estimate bromide, 1-4 ml of the stock was taken in a 25 ml volumetric flask and 2 ml of 9 M sulfuric acid was added and the solution was diluted up to the 25 ml mark. For bromate estimation, 0.1-0.4 ml of the stock was likewise taken in a 25 ml volumetric flask and into it was added a large excess (1 g) of sodium bromide and 2 ml of 9 M sulfuric acid and the volume made up to the 25 ml mark. The liberated bromine as a result of the reaction between bromide and bromate in presence of acid was estimated spectrophotometrically (K. Kumar and D. W. Margerum Inorg. Chem. 1987 26, 2706-2711) by measuring the absorbance at 390 nm and using the appropriate molar extinction coefficient ($\varepsilon$, 167 $M^{-1}$ $cm^{-1}$ in absence and 522 $M^{-1}$ $cm^{-1}$ in the presence of large excess of bromide) values. The homogeneity of solid brominating reagent was confirmed by estimating bromate and bromide composition in 1 g samples drawn from different parts of the sample.

Since the bromide:bromate stoichiometry was always maintained slightly higher than the theoretical ratio of 4: 2 as per the requirement of equation 7, the active bromine was always less than 100 % and was estimated with the help of equation 10:

$$\{3x[BrO_3^-]/([BrO_3^-] + Br^-]\}x100\,\%\,(equation\ 10)$$

The following examples illustrate the method of preparation of the brominating agent and its application in organic bromination reactions.

The important inventive steps involved in the present invention are that (i) the brominating reagent can be prepared from mixed salts of alkali bromide and bromate, either as a solution or solid as desired, (ii) such mixed salt can be prepared cost-effectively from the alkaline bromine intermediate produced in the process of bromine recovery through the "Cold Process", (iii) the ratio of bromide to bromate can be suitably adjusted for aromatic bromination reactions through oxidation with inexpensive hypochlorite which can be prepared, in turn, using chlorine gas and alkali, both of which are used routinely in the "Cold Process", and (iv) the brominating agent can be activated in presence of acid for carrying out organic bromination reactions with high yields and atom efficiency.

One more embodiment of the invention provides a cost effective process for the preparation of a non-hazardous brominating reagent from an aqueous alkaline bromine byproduct solution obtained from bromine recovery plant and containing 25 to 35 % bromine dissolved in aqueous lime or sodium hydroxide containing alkali bromide/bromide mixture having a pH range of 8-12.

Another embodiment of the invention, wherein the aqueous alkaline bromine solution with bromide: bromate ratio typically in the range of 4.5:1 to 5.5:1 and preferably in the range of 5:1 to 5.1:1.

In another embodiment of the invention, wherein the aqueous alkaline bromine solution with bromide: bromate ratio typically in the range of 2:1 to 2.1:1.

Still another embodiment of the invention, wherein bromide to bromate ratio is increased to 5:1 1 to 5.1:1 by adding appropriate quantity of alkali bromide salt to the aqueous alkaline bromine solution.

Yet another embodiment of the invention, wherein bromide to bromate ratio of 2.1:1 to 2:1 is achieved by treating the aqueous alkaline bromine solution with 1-10% concentration of aqueous sodium hypochloride for a period of 6-24 hours at a temperature range of 25-30°C and more preferably by adding alkali into the aqueous alkaline bromine solution and passing chlorine gas to generate hypochloride in situ which can convert bromide salt to bromate salt by the process of oxidation.

Yet another embodiment of the invention, wherein ratio of bromide to bromate is decreased by addition of appropriate quantities of alkaline bromate salt sodium or calcium bromate maintaining the pH of the final solution between 8-10 and without any rise in temperature of the solution.

Yet another embodiment of the invention, wherein the solution of brominating reagent is evaporated by conventional techniques, preferably in solar pans for large scale production to yield solid brominating reagent which is finally ground to get a homogenous mixture of salts having bromine content in the range of 35-70%.

Yet another embodiment of the invention, wherein the said brominating are used to brominate aromatic compounds selected from group comprising bisphenol A, bromophenol, olefins such as styrene, cyclohexene and other class of compounds by activating in situ by the addition of stochiometric quantities of suitable mineral acid and preferably hydrochloric acid.

The following examples are given by way of illustrations and therefore should not be construed to limit the scope of the present invention.

**EXAMPLE 1**

To 9.2 ml of alkaline bromine solution containing 3.03 M Br and 0.64 M $BrO_3^-$, 1.25 mmol (0.129 g) solid NaBr was added to obtain a 5:1 ratio of $Br^-$: $BrO_3^-$. The active bromine content in the mixture was > 99 %.

**EXAMPLE 2** (Comparative)

To 1.818 g (17. 456 mmol) of styrene dissolved in 5ml of dichloromethane in 250 ml round bottom flask, 5ml of 12 N hydrochloric acid and 10 ml of water were added. A mixture of (3.035 g, 29.47 mmol) NaBr and 0.879 g (5.819 mmol) of $NaBrO_3$ dissolved in 20 ml water was added under stirring at room temperature over a period of 90 min. The organic layer was evaporated to get 39.3% of styrene dibromide, 60.6% styrene epoxide and 0.01% benzaldehyde.

**EXAMPLE 3** (Comparative)

0.909 g (8.73 mmol) of styrene dissolved in 5ml of dichloromethane in 250 ml was taken in a round bottom flask fitted with a dropping funnel. In to the dropping funnel, 20 ml of 1.8 M hydrochloric acid and 20 ml of an aqueous solution containing 1.517 g (14.73 mmol) NaBr and 0.439 g (2.91 mmoles) of $NaBrO_3$ were added simultaneously at the same flow rate over a period of 30 min and passage through the dropping funnel outlet insured uniform mixing of the solutions to generate $Br_2$ transiently, as per the reaction of equation 4, before falling into the stirred round bottom flask containing dissolved styrene at room temperature. After completion of addition, stirring was continued for an additional 15 min. The organic layer was then separated and evaporated and the crude solid dissolved in 25 ml methanol at room temperature. 25 ml of water was then added into the methanol and fine white solid separated. The solid which weighed 1.258 g after filtration and drying, was identified as styrene dibromide (mp 68 - 70°C) with isolated yield of 54.6 %.

**EXAMPLE 4** (Comparative)

5.0 g (21.93 mmol) of bisphenol-A, 50 ml of methanol and 18.4 ml of 12 N HCl were taken in a round bottom flask. 15.75 g NaBr (152.9 mmol) and 4.64 g (30.73 mmol) $NaBrO_3$ (were dissolved in 50 ml water and the solution added gradually into the flask over 1.5 h under stirring at room temperature. After completion of the reaction the white crystals that formed at the bottom were filtered and dried to yield 10.68 g (89.5%) of tetrabromobisphenol-A (mp 180 °C). The bromine atom efficiency with respect to desired product was 42.8 %.

**EXAMPLE-5** (Comparative)

62.08 g of sodium hypochlorite was added to the alkaline bromine mixture containing 261.7 g dissolved bromine in sodium hydroxide. The contents were mixed thoroughly and allowed to react for 24 h in a closed 5 L round bottom flask to give brominating reagent containing 241.85 g (92.4%) of active bromine in solution form.

### EXAMPLE 6

0.8 g of sodium hypochlorite was added to the alkaline bromine mixture containing 3.542 g of dissolved bromine in sodium hydroxide. The contents were mixed thoroughly and allowed to react for 12 h in a closed 250 ml round bottom flask. The reaction mixture was evaporated completely on a steam bath. The residue was dried under vacuum to give 9.65 g of solid brominating reagent containing 3.317 g (93.6%) active bromine.

### EXAMPLE 7

12.77 g (0.085 moles) of sodium bromate dissolved in 50 ml of water was added to the alkaline bromine mixture containing 28.54 g of dissolved bromine in sodium hydroxide. The contents were mixed thoroughly for 30 min in a one L flask. The resulting solution was evaporated on a steam bath and dried under vacuum to give 67 g of solid brominating reagent containing 35.095 g (99.4%) of active bromine.

### EXAMPLE 8

1 g (4.386 mmol) of bisphenol-A, 4.075 g of the brominating agent of Example 6 dissolved in 14.5 ml water (containing 17.511 mmol of reactive bromine) and 5 ml of dichloromethane were taken in a round bottom flask and a solution of 12 ml of 2 N hydrochloric acid was added over a period of 40 min under stirring at room temperature. Stirring was continued for another 30 min. 1.55 g of fine crystals were obtained on filtration and a further 0.67 g was obtained after evaporation of the organic layer to give a total product amount of 2.22 g (93 .2%) tetrabromobisphenol-A (mp 176-182 °C) characterized by spectroscopic techniques. The bromine atom efficiency based on desired product and active bromine content was 93.2 %.

### EXAMPLE 9 (Comparative)

4.3 ml of alkaline (lime) bromine (containing 0.242 g bromide and 0.051 g bromate per ml of solution) and 0.52 g sodium bromate in 10 ml water were taken in a round bottom flask with total bromine content of 19.135 mmol. Into this was added 2.5 g (18.517 mmol) of acetanilide in 12.5 ml dichloromethane. 25 ml of 3.6 N hydrochloric acid was added over a period of 13 min under stirring at room temperature. Stirring was continued for another 30 min. The precipitate was filtered, washed with water and dried to give 3.689 g (92.7 %) of p-bromoacetanilide (m.p. 164-168 °C) characterized through spectroscopic techniques.

### EXAMPLE 10

To 2.5 gm (26.596 mmoles) of phenol dissolved in a mixture of 30 ml water and 9 ml of 12 N hydrochloric acid, 18.568 g of the brominating agent of Example 6 (containing 79.788 mmol of reactive bromine) dissolved in 66 ml water was added over a period of 60 min under stirring at room temperature. Stirring was continued for another 30 min. The precipitate was filtered, washed with water and dried to give 8.495 g (96.5%) of tribromophenol (mp 84-89 °C) which was characterized through spectroscopic techniques.

### EXAMPLE 11

1 g (7.407 mmol) of acetanilide, 1.724 g of the brominating agent of Example 6 dissolved in 6.2 ml water (containing 7.408 mmol of reactive bromine) and 5 ml of dichloromethane were taken in a round bottom flask and a a solution of 12 ml of 2 N hydrochloric acid was added over a period of 15 min under stirring at room temperature. Stirring was continued for another 30 min. The precipitate was filtered, washed with water and dried to give 1.402 g (88 %) of p-bromoacetanilide (m.p. 164-168 °C) characterized through spectroscopic techniques.

**The main advantages of the present invention are:**

(i) Inexpensive method of preparation of non-hazardous brominating reagents that are stable under storage and can be formulated either in solution or solid forms.

(ii) The brominating reagents can be prepared as a mixture of alkali bromide and bromate salts by utilizing the aqueous alkaline bromine mixture produced as intermediate in bromine recovery plants based on the Cold Process.

(iii) The alkaline bromine mixture with 5:1 bromide: bromate is a substitute for liquid bromine in bromine addition reactions and can be used for aromatic bromination reactions.

(iv) The brominating reagent with 2:1 bromide: bromate can be prepared from the alkaline bromine mixture through oxidation with inexpensive oxidizing agents and is especially suitable for aromatic bromine reactions with high Br atom efficiency that avoids the formation of HBr.

(v) The brominating reagents are activated by simple addition of mineral acid and no catalyst is required for the bromination reactions.

(vi) Bromination reaction can be carried out with the present brominating reagents under ambient conditions.

## Claims

1. A stable non-hazardous brominating reagent from an aqueous alkaline bromine byproduct solution obtained from bromine recovery plant, said bromine byproduct solution containing 25 to 35 % bromine dissolved in aqueous lime or sodium hydroxide in the form of alkali bromide and alkali bromate mixture, said reagent having a pH ranging between 8.5 - 10.5 and containing 100 - 350 g/L bromine or in a solid form having 45-55% (w/w) bromine.

2. A cost-effective process for the preparation of a stable and non-hazardous brominating reagent, which comprises treating aqueous alkaline bromine containing 25-35 % bromine dissolved in aqueous lime or sodium hydroxide as an alkali bromide and alkali bromate mixture at a pH ranging between 8-12 to increase the stoichiometric ratio of bromide to bromate in the range of 5.1:1 to 5:1 or adding an oxidizing agent to decrease the stoichiometric ratio of bromide to bromate in the range of 2.1:1 to 2:1 to obtain the desired brominating reagent solution having pH ranging between 8.5-10.5 and containing 100-350 g/L bromine, optionally evaporating the above said brominating reagent solution to obtain the desired brominating reagent in a solid form possessing 45-55 % (w/w) bromine.

3. A process as claimed in claim 2, wherein the bromide to bromate ratio is increased to 5:1 by adding appropriate quantity of alkali bromide salt to the aqueous alkaline bromine solution.

4. A process as claimed in claim 2, wherein the bromide to bromate ratio of 2:1 is achieved by treating the aqueous alkaline bromine solution with 1-10% concentration of aqueous sodium hypochloride for a period of 6-24 hours at a temperature in the range of range of 25-30°C and more preferably by adding alkali into the aqueous alkaline bromine solution followed by passing chlorine gas to generate hypochloride in situ to convert bromide salt to bromate salt by an oxidation process.

5. A process as claimed in claim 2, wherein the bromide to bromate ratio of alkaline bromine mixture is decreased by adding appropriate quantity of alkaline bromate salt, preferably sodium or calcium bromate while ensuring that pH of the solution remains between 8-10 and there is no rise in temperature.

6. A process as claimed in claim 2, wherein the solution of brominating reagent is evaporated by conventional techniques, preferably in solar pans for large scale production to obtain the solid brominating reagent which is finally ground to get a homogenous mixture of salts having bromine content in the range of 90-100%.

7. A process as claimed in claim 2, wherein the said brominating reagent is activated *in situ* during the bromination reaction through addition of stoichiometric quantity of a mineral acid, preferably hydrochloric acid.

8. A method for brominating aromatic compounds by using brominating reagent as claimed in claim 1, wherein the aromatic compound used is selected from group consisting of bisphenol A, bromophenol and olefin selected from styrene and cyclohexene and other class of aromatic compounds and, said method comprising: treating aqueous alkaline bromine containing 25-35 % bromine dissolved in aqueous lime or sodium hydroxide as an alkali bromide and alkali bromate mixture at a pH ranging between 8-12 with alkali bromide to increase the stoichiometric ratio of bromide to bromate in the range of 5.1:1 1 to 5:1 or adding an oxidizing agent to decrease the stoichiometric ratio of bromide to bromate in the range of 2.1:1 to 2:1 to obtain the desired brominating reagent solution having pH ranging between 8.5-10.5 and containing 100-350 g/L bromine, optionally evaporating the above said brominating reagent solution to obtain the desired brominating reagent in a solid form possessing 45-55 % (w/w) bromine with active bromine content that is 90-100 % of total bromine content and activating the brominating agent *in situ* by the addition of mineral acid,

9. The method as claimed in claim 8, wherein the mineral acid is selected from hydrochloric acid or sulfuric acid and preferable hydrochloric acid.

10. The method as claimed in claim 9, wherein the quantity of 12N hydrochloric acid used is 9 to 12 ml to activate 1 -20 g of said brominating agent to brominate 1-3 g of an organic substrate.

**Patentansprüche**

1. Stabiles, ungefährliches Bromierungsmittel aus einer wässrigen alkalischen, von einer Bromrückgewinnungsanlage gewonnenen Bromabfalllösung wobei die Bromabfalllösung, aufgelöst in Gestalt einer Mischung von Alkalibromid und Alkalibromat in wässrigem Calcium- oder Natriumhydroxid, 25 bis 35 % Brom enthält und wobei das Mittel einen pH-Wert im Bereich von 8,5 bis 10,5 besitzt und 100 bis 350 g/l Brom enthält oder in fester Form 45 bis 55 % (Gew./Gew.) an Brom besitzt.

2. Kostengünstiger Prozess für die Herstellung eines stabilen und ungefährlichen Bromierungsmittels, der umfasst, eine wässrige alkalische Bromlösung, die 25 bis 35 % Brom als Mischung aus Alkalibromid und Alkalibromat in wässrigem Calcium- oder Natriumhydroxid gelöst enthält, bei einem pH-Wert im Bereich von 8 bis 12 zu behandeln, um das stöchiometrische Verhältnis von Bromid zu Bromat in den Bereich von 5,1 : 1 bis 5 : 1 zu erhöhen, oder ein Oxidationsmittel hinzuzufügen, um das stöchiometrische Verhältnis von Bromid zu Bromat in den Bereich von 2,1 : 1 bis 2 : 1 abzusenken, um die gewünschte Bromierungsmittellösung zu gewinnen, die einen pH-Wert im Bereich von 8,5 bis 10,5 besitzt und 100 bis 350 g/l Brom enthält, und wahlweise die obige Bromierungsmittellösung einzudampfen, um das gewünschte Bromierungsmittel in einer festen Form mit 45 bis 55 % (Gew./Gew.) Brom zu gewinnen.

3. Prozess nach Anspruch 2, **dadurch gekennzeichnet, dass** das Verhältnis von Bromid zu Bromat durch Zusatz einer geeigneten Menge von Alkalibromidsalz zur wässrigen alkalischen Bromlösung auf 5 : 1 erhöht wird.

4. Prozess nach Anspruch 2, **dadurch gekennzeichnet, dass** das Verhältnis von Bromid zu Bromat von 2 : 1 erreicht wird, indem die wässrige alkalische Bromlösung während einer Zeitdauer von 6 bis 24 Stunden bei einer Temperatur im Bereich von 25 bis 30 °C mit 1- bis 10-prozentigem wässrigem Natriumhypochlorid behandelt wird, und stärker bevorzugt, indem der wässrigen alkalischen Bromlösung Alkali hinzugefügt wird und danach Chlorgas durchgeleitet wird, um Hypochlorid in situ zu erzeugen und durch einen Oxidationsprozess Bromidsalz in Bromatsalz umzuwandeln.

5. Prozess nach Anspruch 2, **dadurch gekennzeichnet, dass** das Verhältnis von Bromid zu Bromat in der alkalischen Brommischung abgesenkt wird, indem eine geeignete Menge von alkalischem Bromatsalz hinzugefügt wird, bevorzugt Natrium- oder Calciumbromat, während dafür gesorgt wird, **dass** der pH-Wert der Lösung zwischen 8 und 10 bleibt und kein Temperaturanstieg auftritt.

6. Prozess nach Anspruch 2, **dadurch gekennzeichnet, dass** die Bromierungsmittellösung mit herkömmlichen Verfahren eingedampft wird, bevorzugt in Solarpfannen zur Produktion im Grossmassstab, um das feste Bromierungsmittel zu gewinnen, das fein gemahlen wird, um ein homogenes Gemisch von Salzen zu erlangen, die einen Bromgehalt im Bereich von 90 bis 100 % besitzen.

7. Prozess nach Anspruch 2, **dadurch gekennzeichnet, dass** das Bromierungsmittel durch Zugabe einer stöchiometrischen Menge von Mineralsäure, bevorzugt von Chlorwasserstoffsäure, während der Bromierungsreaktion in situ aktiviert wird.

8. Verfahren zur Bromierung aromatischer Verbindungen unter Verwendung eines Bromierungsmittels nach Anspruch 1, **dadurch gekennzeichnet, dass** die verwendete aromatische Verbindung aus der Gruppe von Bisphenol A, Bromphenol und einem aus Styrol und Cyclohexen ausgewählten Olefin und anderen Klassen aromatischer Verbindungen ausgewählt wird und das Verfahren umfasst: die wässrige alkalische Bromlösung mit 25 bis 35 % Brom, aufgelöst als eine Mischung von Alkalibromid und Alkalibromat in wässriger Calcium- oder Natriumhydroxidlösung, bei einem pH-Wert im Bereich von 8 bis 12 mit Alkalibromid zu behandeln, um das stöchiometrische Verhältnis vom Bromid zu Bromat in den Bereich von 5,1 : 1 bis 5 : 1 zu erhöhen, oder ein Oxidationsmittel zuzusetzen, um das stöchiometrische Verhältnis von Bromid zu Bromat in den Bereich von 2,1 : 1 bis 2 : 1 abzusenken, um die gewünschte Bromierungsmittellösung zu gewinnen, die einen pH-Wert im Bereich zwischen 8,5 und 10,5 besitzt und 100 bis 350 g/l Brom enthält, wahlweise die genannte Bromierungsmittellösung einzudampfen, um das gewünschte Bromierungsmittel in einer festen Gestalt zu gewinnen, die 45 bis 55 % (Gew./Gew.) Brom bei einem aktiven Bromgehalt von 90 bis 100 % des gesamten Bromgehalts besitzt, und das Bromierungsmittel durch Zusatz von Mineralsäure in situ zu aktivieren.

9. Verfahren nach Anspruch 8, **dadurch gekennzeichnet, dass** die Mineralsäure aus Chlorwasserstoffsäure oder Schwefelsäure ausgewählt wird und vorzugsweise Chlorwasserstoffsäure ist.

**10.** Verfahren nach Anspruch 9, **dadurch gekennzeichnet, dass** die verwendete Menge an 12 N Chlorwasserstoffsäure 9 bis 12 ml beträgt, um 1 bis 20 g des Bromierungsmittels zu aktivieren, damit 1 bis 3 g eines organischen Substrats bromiert werden.

## Revendications

**1.** Un réactif bromé non-dangereux stable à partir d'une solution de sous-produit de brome alcaline aqueuse obtenue par une usine de récupération de brome, ladite solution de sous-produit de brome contenant le brome de 25 à 35 % dissous dans de la chaux aqueuse ou de l'hydroxyde de sodium en forme de bromure d'alcali et de mélange d'alcali bromé, ledit réactif ayant un pH s'étendant entre 8.5-10.5 et contenant de 100-350 g/L de brome ou en forme solide ayant 45-55 % (p/p) de brome.

**2.** Un processus avantageux de préparation d'un réactif bromé stable et non-dangereux, qui comprend du brome alcalin aqueux traité contenant 25-35 % du brome dissous dans de la chaux aqueuse ou de l'hydroxyde de sodium sous forme de bromure d'alcali et un mélange d'alcali bromé à un pH compris entre 8 - 12 en augmentant le ratio stoechiométrique de bromure de brome dans la gamme de 5.1:1 à 5:1 ou en ajoutant un agent oxydant pour diminuer le ratio stoechiométrique de bromure de brome dans la gamme de 2,1:1 à 2:1 pour obtenir la solution réactive bromé souhaité ayant un pH compris entre 8.5-10.5 et contenant 100-350 g / L de brome, optionnellement évaporer ladite solution du réactif bromé pour obtenir le réactif bromé souhaité dans une forme solide possédant 45-55% (p/p) de brome.

**3.** Un procédé selon la revendication 2, dans lequel le ratio de bromure de brome est passé à 5:1 en ajoutant la quantité appropriée de bromure de sel alcalin à la solution bromée alcaline aqueuse.

**4.** Un procédé selon la revendication 2, dans lequel le ratio de bromure de brome de 2:1 est obtenu par le traitement de la solution bromée alcaline aqueuse avec 1-10% de concentration de sodium hypochloride aqueux durant une période de 6-24 heures à une température comprise entre 25-30 °C et de préférence en ajoutant l'alcali dans la solution bromée aqueuse en solution alcaline suivie par une étape par un gaz chloré pour produire l'hypochloride in situ et convertir le sel de bromure en sel bromé par un processus d'oxydation.

**5.** Un procédé selon la revendication 2, dans lequel le ratio de bromure de brome du mélange de brome alcalin est diminué par l'ajout de quantité appropriée de sel de brome alcalin, de préférence de sodium ou de calcium bromé, en veillant à ce que le pH de la solution reste entre 8 - 10 et qu'il n'y ait pas d'augmentation de température.

**6.** Un procédé selon la revendication 2, dans lequel la solution du réactif bromé est évaporé par des techniques conventionnelles, de préférence dans des fours solaires pour la production à grande échelle pour obtenir le réactif solide bromé qui est finalement déposé pour obtenir un mélange homogène de sels ayant contenu de brome dans le gamme de 90-100%.

**7.** Un procédé selon la revendication 2, par lequel ledit réactif bromé est activé in situ au cours de la réaction de bromination par l'ajout d'une quantité stoechiométrique d'un acide minéral, de préférence l'acide chlorhydrique.

**8.** Une méthode pour bromer des composés aromatiques par l'utilisation de réactif bromé selon la revendication 1, par laquelle le composé aromatique utilisé est sélectionné depuis le groupe composé de bisphénol A, bromophénol et oléfine choisis depuis le styrène et le cyclohexène et les autres classes de composés aromatiques, ladite méthode comprenant:

- le traitement du brome alcalin aqueux traité contenant 25-35 % du brome dissous dans de la chaux aqueuse ou de l'hydroxyde de sodium sous forme de bromure d'alcali et un mélange d'alcali bromé à un pH compris entre 8 - 12 pour augmenter le ratio stoechiométrique de bromure de brome dans la gamme de 5.1:1 à 5:1 ou en ajoutant un agent oxydant pour diminuer le ratio stoechiométrique de bromure de brome dans la gamme de 2,1:1 à 2:1 pour obtenir la solution réactive bromé souhaité ayant un pH compris entre 8.5-10.5 et contenant 100-350 g/L de brome, optionnellement évaporer ladite solution du réactif bromé pour obtenir le réactif bromé souhaité dans une forme solide possédant 45-55% (p/p) de brome avec le contenu du brome actif qui représente entre 90-100% du total du contenu du brome et l'activation de l'agent bromé in situ par l'ajout d'acide minéral.

**9.** La méthode selon la revendication 8, par laquelle l'acide minéral est choisi à partir de l'acide chlorhydrique ou sulfurique, de préférence l'acide chlorhydrique.

10. La méthode selon la revendication 9, par laquelle la quantité d'acide chlorhydrique 12N utilisée est de 9 à 12 ml pour activer 1- 20 g de bromure dudit agent bromé pour bromer 1- 3 g d'un substrat organique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5475153 A

- US 09767667 B

**Non-patent literature cited in the description**

- **C. A. BUECHLER ; D. E. PEARSON.** Survey of organic syntheses. Wiley -Inter science, 1970
- **A. GROWEISS.** *Organic Process & Development,* 2000, vol. 4, 30-33
- **P. C. MERKER ; J. A. VONA.** *J Chem. Ed.,* 1949, vol. 26, 613
- **L. F. FIESER ; M. FIESER.** Reagents for Organic Chemistry. John Wiley, 1967, vol. 1, 967
- **G. ROTHENBERG ; J. H. CLARK.** *Organic Process & Development,* 2000, vol. 4, 270-274
- **K. KUMAR ; D. W. MARGERUM.** *Inorg. Chem.,* 1987, vol. 26, 2706-2711